(19) **Europäisches Patentamt European Patent Office Office européen des brevets**

(11) **EP 3 130 232 A1**

(12) **EUROPEAN PATENT APPLICATION**
published in accordance with Art. 153(4) EPC

(43) Date of publication:
**15.02.2017 Bulletin 2017/07**

(21) Application number: **15773190.2**

(22) Date of filing: **31.03.2015**

(51) Int Cl.:
**A23L 2/38** (2006.01)     **A23L 33/105** (2016.01)
**A23L 33/16** (2016.01)

(86) International application number:
**PCT/KR2015/003188**

(87) International publication number:
**WO 2015/152615 (08.10.2015 Gazette 2015/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **31.03.2014 KR 20140038070**

(71) Applicant: **Aribio Inc.**
**Seoul 150-095 (KR)**

(72) Inventors:
 • **CHOUNG, Jai Jun**
   **Yongin-si**
   **Gyeonggi-do 448-132 (KR)**

 • **SUNG, Soo Hyun**
   **Seoul 134-050 (KR)**
 • **KU, Sae Kwang**
   **Daegu 706-920 (KR)**
 • **LEE, Young Joon**
   **Daegu 700-082 (KR)**
 • **KIM, Jong Kyu**
   **Yongin-si**
   **Gyeonggi-do 446-954 (KR)**
 • **SHIN, Chang Ho**
   **Ansan-si**
   **Gyeonggi-do 425-724 (KR)**

(74) Representative: **Engelhard, Markus**
   **Boehmert & Boehmert**
   **Anwaltspartnerschaft mbB**
   **Patentanwälte Rechtsanwälte**
   **Pettenkoferstrasse 20-22**
   **80336 München (DE)**

(54) **FUNCTIONAL BEVERAGE INCLUDING HIGH HARDNESS MINERAL WATER PRODUCED FROM SALTY UNDERGROUND WATER OR DEEP SEAWATER**

(57)     The present invention relates to a functional beverage including high hardness mineral water produced from salty underground water or deep seawater as an effective ingredient. The functional beverage of the present invention may not only impart a refreshing sensation in the mouth upon consumption, but may also effectively remove fatigue-causing substances such as lactic acid and free oxygen radicals and increase ATP production in the body, and vitalize one's lifestyle and improve exercise capacity and cognitive functions. The functional beverage of the present invention may show the effects of improving or maintaining anti-inflammatory and immune functions.

**Fig. 2**

**Change in ATP level in whole blood**

**EP 3 130 232 A1**

**Description**

Technical Field

The present invention relates to a functional beverage containing, as an active ingredient, high-hardness mineral water prepared from salty underground water or deep ocean water.

Background Art

Modern people spend their lives complaining of various symptoms, such as chronic deterioration of physical strength or deterioration of immunity, due to busy mental and physical activities, too much stress, or the like, caused by rapid changes in the lifestyle pattern and environment. In order to overcome these problems, the development of functional beverages, such as invigorating beverages and fatigue-recovering beverages, is proceeding actively, and these products are increasingly being sold in the market.

In addition, modern living facilities have significantly improved compared with the past due to economic growth, but the living environment gets worse and worse due to the severe contamination of the environment, such as air and water quality, and modern people are getting more overweight due to excessive intake of high-calorie foods and lack of exercise, and are vulnerable to various diseases due to acidified physical constitution, but satisfactory solutions therefor are not actually provided.

Due to these facts, there is an increasing need for functional beverages that can be conveniently purchased from nearby shops or the like, convenient to carry, give vigor to the body, like health supplement foods, and can effectively remove fatigue-inducing materials, such as lactic acid and reactive oxygen species, in the body.

Athletes drink sports beverages for the regulation of body fluid and the maintenance of a balanced electrolyte concentration through fast rehydration for dehydration caused by endurance exercise for a long time, but do not obtain any particular effect in view of the improvement in exercise ability or fatigue recovery. Furthermore, high concentrations of sugars and sodium contained in the sports beverages may negatively influence the health of non-athletes participating in leisure activities, and thus the development of functional beverages that have exercise-functionality improved efficiency and may positively influence health is urgently needed.

Throughout the entire specification, many papers, and patent documents are referenced and their citations are represented. The disclosure of the cited papers and patent documents are entirely incorporated by reference into the present specification, and the level of the technical field within which the present invention falls and the details of the present invention are explained more clearly.

Detailed Description of the Invention

Technical Problem

The present inventors searched for and endeavored to develop a multi-functional beverage that gives a feeling of refreshment in the mouth if drank, effectively removes fatigue-inducing materials, such as lactate and reactive oxygen species, and increases ATP production in the body, thus ultimately giving vigor to daily life through fatigue recovery, improves exercise ability and cognitive functions, and exhibits an anti-inflammatory effect and an immune dysfunction improving effect. As a result, the present inventors verified, through clinical experiments using high-hardness mineral water prepared from salty underground water or deep ocean water, that the intake of the high-hardness mineral water can obtain a body function promotion effect, such as increasing ATP production in the body, increasing energy metabolism in the body, reducing reactive oxygen species in the body, increasing minerals in the body, suppressing lactate accumulation, reducing the fatigue degree, improving exercise ability and cognitive functions, having an anti-inflammation effect, and maintaining immune functions or improving immune dysfunctions, and thus completed the present invention.

Therefore, an aspect of the present invention is to provide a functional beverage (or functional beverage composition) containing, as an active ingredient, high-hardness mineral water prepared from salty underground water or deep ocean water.

Another aspect of the present invention is to provide a method for improving body functions by administering the functional beverage to a subject.

Still another aspect of the present invention is to provide a use of mineral water having a hardness value of 100-2,000, prepared from salty underground water or deep ocean water, for preparing the functional beverage.

Other purposes and advantages of the present disclosure will become more obvious with the following detailed description of the invention, claims, and drawings.

Technical Solution

In accordance with an aspect of the present invention, there is provided a functional beverage containing, as an active ingredient, mineral water having a hardness value of 100-2,000 prepared from salty underground water or deep ocean water.

In accordance with another aspect of the present invention, there is provided a method for improving body functions, the method including administering, to a subject, a functional beverage containing, as an active ingredient, mineral water having a hardness value of 100-2,000 prepared from salty underground water or deep ocean water, wherein the improving of the body functions is selected from the group consisting of increasing ATP production in the body, increasing energy metabolism in the body, increasing antioxidative activity in the body, increasing minerals in the body, suppressing lactate accumulation, improving exercise ability, reducing or recovering from fatigue, preventing muscular pain, improving cognitive functions, having an anti-inflammation effect, and maintaining immune functions or improving immune dysfunctions.

In accordance with still another aspect of the present invention, there is provided a use of the mineral water for preparing a functional beverage, the mineral water having a hardness value of 100-2,000 prepared from salty underground water or deep ocean water.

The present inventors searched for and endeavored to develop a multi-functional beverage that gives a feeling of refreshment in the mouth if drank, effectively removes fatigue-inducing materials, such as lactate and reactive oxygen species, and increases ATP production in the body, thus ultimately giving vigor to daily life through fatigue recovery, improves exercise ability and cognitive functions, and exhibits an anti-inflammatory effect and an immune dysfunction improving effect. As a result, the present inventors verified, through clinical experiments using high-hardness mineral water prepared from salty underground water or deep ocean water, that the intake of the high-hardness mineral water can obtain a body function promotion effect, such as increasing ATP production in the body, increasing energy metabolism in the body, reducing reactive oxygen species in the body, increasing minerals in the body, suppressing lactate accumulation, reducing the fatigue degree, improving exercise ability and cognitive functions, having an anti-inflammation effect, and maintaining immune functions or improving immune dysfunctions.

As used herein, the term "salty underground water" refers to underground water in bedrock aquifers containing more than predetermined amounts of dissolved solids, such as salt, and means raw water which is to be drunk in a natural state in which the water quality can be continuously maintained, and the term "deep ocean water" refers to water in the sea that is at a depth of 200 m or deeper, where sunlight does not reach.

The high-hardness mineral water, as an active ingredient, contained in the functional beverage of the present invention may be prepared using salty underground water, and any salty underground water may be used in the preparation of the high-hardness mineral water without limitation. For example, high-hardness mineral water may be prepared by using underground water in bedrock aquifers, which has a totally dissolved solid content of 2,000 mg/ℓ, including a salt dissolved in the water.

As used herein, the term "hardness" refers to water strength which is generated by divalent metal cations, such as $Ca^{2+}$ and $Mg^{2+}$, dissolved in water, and expressed as a value in terms of $CaCO_3$.

According to an embodiment of the present invention, the hardness may be calculated by the following equation

$$[\text{Equation 1}]$$
$$\text{Water hardness } (CaCO_3 \text{ mg/}\ell) = 2.5 \times [Ca^{2+} \text{ concentration (mg/}\ell)] + 4.1 \times [Mg^{2+} \text{ concentration (mg/}\ell)]$$

The mineral water of the present invention has a hardness (mg/ℓ as $CaCO_3$) of 100-2,000. The mineral water with a hardness of 100-2,000 may contain 15-500 mg/ℓ magnesium, 5-170 mg/ℓ calcium, and 4.5-150 mg/ℓ potassium.

The mineral water of the present invention may have various hardness values within the above hardness range. According to an embodiment of the present invention, the hardness of the mineral water is 100-1900, 100-1800, 100-1700, 100-1600, 100-1500, 100-1400, 100-1300, 100-1200, 100-1100, or 100-1000; according to another embodiment, the hardness of the mineral water is 200-1900, 200-1800, 200-1700, 200-1600, 200-1500, 200-1400, 200-1300, 200-1200, 200-1100, or 200-1000; according to still another embodiment, the hardness of the mineral water is 300-1900, 300-1800, 300-1700, 300-1600, 300-1500, 300-1400, 300-1300, 300-1200, 300-1100, or 300-1000; according to still another embodiment, the hardness of the mineral water is 400-1900, 400-1800, 400-1700, 400-1600, 400-1500, 400-1400, 400-1300, 400-1200, 400-1100, or 400-1000; according to still another embodiment, the hardness of the mineral water is 500-1900, 500-1800, 500-1700, 500-1600, 500-1500, 500-1400, 500-1300, 500-1200, 500-1100, or 500-1000; according to still another embodiment, the hardness of the mineral water is 600-1900, 600-1800, 600-1700, 600-1600, 600-1500, 600-1400, 600-1300, 600-1200, 600-1100, or 600-1000.

Of the mineral water having the above hardness values, for a representative example, the mineral water with a hardness of 100 contains 15-25 mg/ℓ magnesium, 5-8.5 mg/ℓ calcium, and 4.5-7.5 mg/ℓ potassium; the mineral water with a hardness of 300 contains 45-75 mg/ℓ magnesium, 15-25.5 mg/ℓ calcium, and 13.5-22.5 mg/ℓ potassium; the mineral water with a hardness of 700 contains 105-175 mg/ℓ magnesium, 35-59.5 mg/ℓ calcium, and 31.5-52.5 mg/ℓ potassium; and the mineral water with a hardness of 1000 contains 150-250 mg/ℓ magnesium, 50-85 mg/ℓ calcium, and 45-75 mg/ℓ potassium (containing 15-25 mg/ℓ magnesium, 5-8.5 mg/ℓ calcium, and 4.5-7.5 mg/ℓ potassium per hardness of 100).

According to an embodiment of the present invention, the functional beverage of the present invention includes mineral water with a hardness of 100-1200 containing 15-300 mg/ℓ magnesium, 5-102 mg/ℓ calcium, and 4.5-90 mg/ℓ potassium.

According to an embodiment of the present invention, the functional beverage of the present invention includes mineral water with a hardness of 200-1200 containing 30-300 mg/ℓ magnesium, 10-102 mg/ℓ calcium, and 9-90 mg/ℓ potassium.

According to an embodiment of the present invention, the functional beverage of the present invention includes mineral water with a hardness of 300-1200 containing 45-300 mg/ℓ magnesium, 15-102 mg/ℓ calcium, and 13.5-90 mg/ℓ potassium.

According to another embodiment of the present invention, the functional beverage of the present invention includes mineral water with a hardness of 400-1200 containing 60-300 mg/ℓ magnesium, 20-102 mg/ℓ calcium, and 18-90 mg/ℓ potassium.

According to still another embodiment of the present invention, the functional beverage of the present invention includes mineral water with a hardness of 500-1200 containing 75-300 mg/ℓ magnesium, 25-102 mg/ℓ calcium, and 22.5-90 mg/ℓ potassium.

According to still another embodiment of the present invention, the functional beverage of the present invention includes mineral water with a hardness of 600-1200 containing 90-300 mg/ℓ magnesium, 30-102 mg/ℓ calcium, and 27-90 mg/ℓ potassium.

The functional beverage of the present invention exhibits several functionalities useful to the body of a drinker. Therefore, the functional beverage of the present invention is a functional beverage for improving body functions. The functionality or the improvement of body functions that can be achieved through the intake of the functional beverage of the present invention includes increasing ATP production in the body, increasing energy metabolism in the body, increasing antioxidative activity in the body, increasing minerals in the body, suppressing lactate accumulation, improving the exercise ability, reducing or recovering from fatigue, preventing muscular pain, improving cognitive functions, having an anti-inflammation effect, and maintaining immune functions or improving immune dysfunctions.

According to an embodiment of the present invention, the present invention is a functional beverage for increasing ATP production in the body or increasing energy metabolism in the body. For example, such a functional beverage can be drunk in order to improve exercise ability or recover from fatigue. In these cases, the hardness value of the mineral water contained in the functional beverage of the present invention may be 300-900. The hardness value of the mineral water is 350-850 for a particular embodiment, 400-800 for another embodiment, 450-800 for still another embodiment, 500-800 for still another embodiment, 550-800 for still another embodiment, 600-800 for still another embodiment, and 650-800 for still another embodiment.

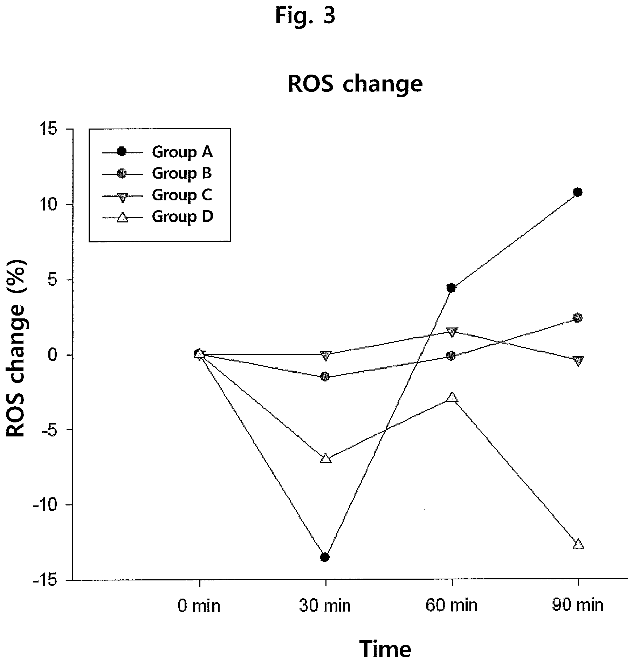

According to another embodiment of the present invention, the present invention is a functional beverage for increasing antioxidative activity (reducing reactive oxygen species) in the body. For example, the reactive oxygen species are representative fatigue-inducing materials, and thus, for the prevention, reduction, and/or recovery from fatigue, the functional beverage of the present invention having an antioxidative activity can be drunk (see FIG. 3). In these cases, the hardness value of the mineral water contained in the functional beverage of the present invention may be 600-1500. The hardness value of the mineral water is 600-1400 for a particular embodiment, 600-1300 for another particular embodiment, 600-1200 for still another particular embodiment, and 650-1100 for still another particular embodiment.

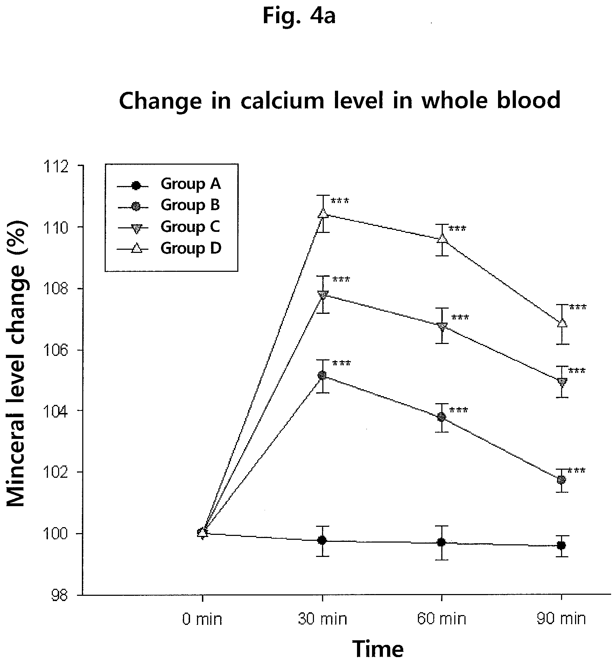

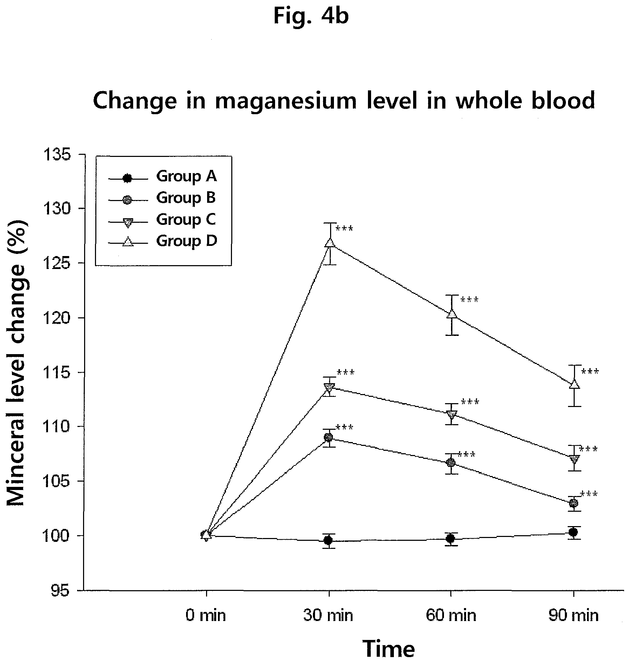

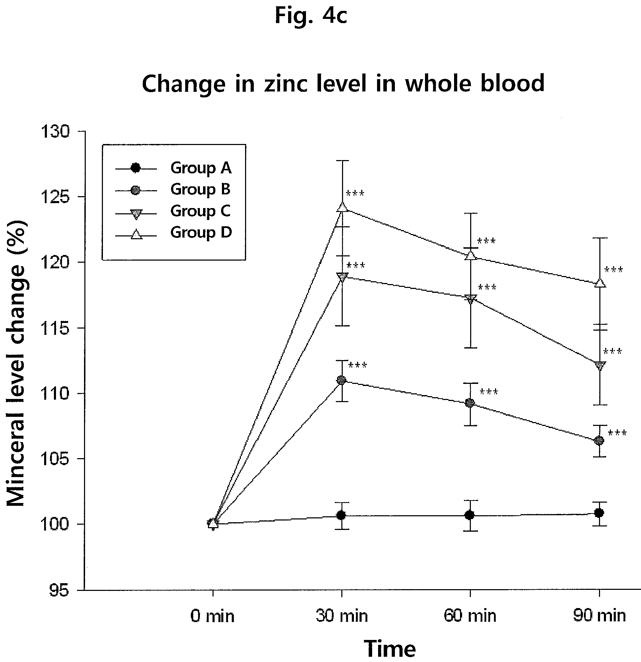

According to still another embodiment of the present invention, the present invention is a functional beverage for supplying or supplementing minerals in the body. For example, for the supply and supplement of various minerals (e.g., calcium, magnesium, potassium, and zinc), such a functional beverage can be drank (see FIGS. 4a-4c). Especially, although the functional beverage of the present invention contains a very slight amount of zinc compared with the other minerals, the level of zinc in the body of the functional beverage drinker can be significantly increased (see FIG. 4). Zinc, which is a structural ingredient in over 200 kinds of *in vivo* enzymes, is involved in main metabolic procedures or reactions in the body, and has been known to be effective for, especially, immunity enhancement, hair loss prevention and treatment, acne treatment, and prostate health promotion. Therefore, the supply of zinc by drinking the functional beverage of the present invention can prevent the deficiency of zinc and obtain effects of improving zinc-related body functions, such as immunity enhancement.

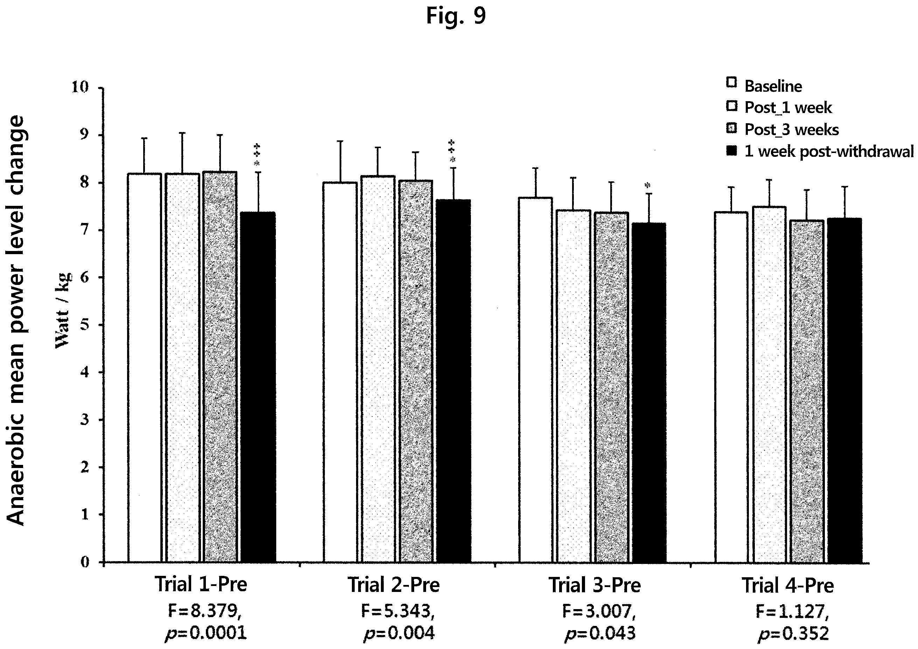

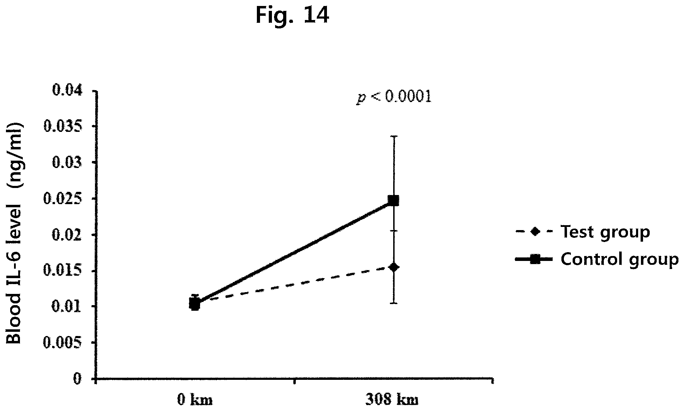

According to still another embodiment of the present invention, the present invention is directed to a functional beverage for improving exercise ability. The intake of the functional beverage can improve various types of exercise ability, such muscular strength, endurance (muscular endurance, cardiovascular endurance, etc.), quickness, and exercise concentration ability (see PCT International application and FIG. 9). Furthermore, the intake of the functional beverage can suppress physical stress and the resultant inflammation responses due to the long-term exercise (physical activity) and can also prevent immune dysfunctions (see FIGs. 14 to 16).

In a particular embodiment, the improvement of exercise ability is selected from the group consisting of increasing ATP production in the body, increasing energy metabolism in the body, suppressing lactate accumulation, reducing or recovering from fatigue, preventing muscular pain, improving cognitive functions, suppressing inflammation resulting from physical activity, and improving immune dysfunctions resulting from physical activity.

According to still another embodiment of the present invention, the present invention is directed to a functional beverage for reducing or recovering from fatigue.

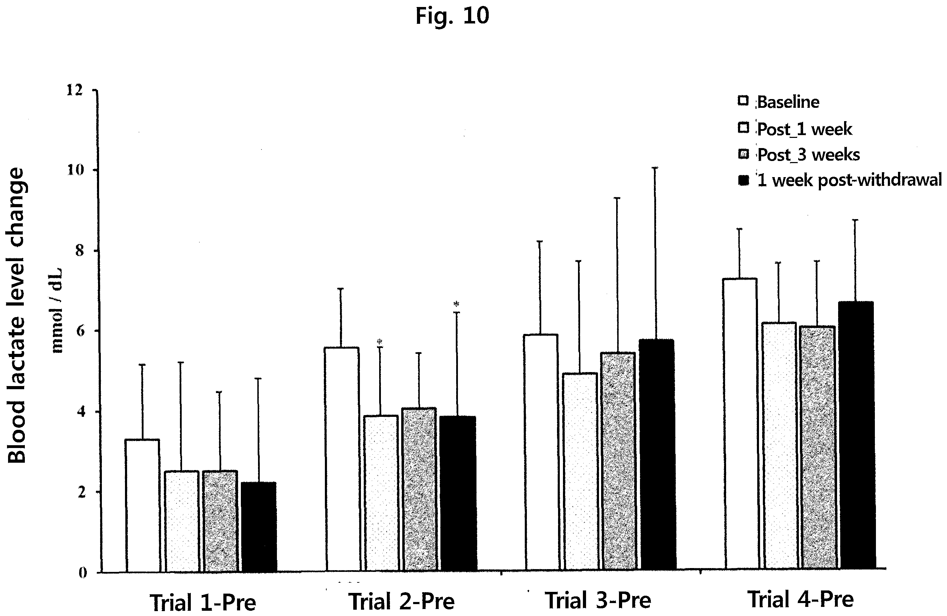

As used herein, the term "fatigue" refers to the deterioration of physical functions caused by consecutive and repetitive mental and physical work, and exercise fatigue due to physical fatigue and lactate accumulation. In cases of severe physical activity, such as exercise and physical labor, glycogen, which is an energy source, is decomposed due to a lack of oxygen, to generate lactic acid, resulting in the feeling of fatigue. The functional beverage of the present invention suppresses lactate accumulation (see FIG. 10) and exhibits an antioxidative effect of removing reactive oxygen species, which are fatigue-inducing materials (see FIG. 3), thereby showing excellent effects of preventing muscular pain, and recovering from fatigue.

According to still another embodiment of the present invention, the present invention is directed to a functional beverage for preventing muscular pain. The lactate accumulation due to excessive physical exercise causes muscular pain. The functional beverage of the present invention suppresses lactate accumulation (see FIG. 10), thereby exhibiting a preventive effect against muscular pain.

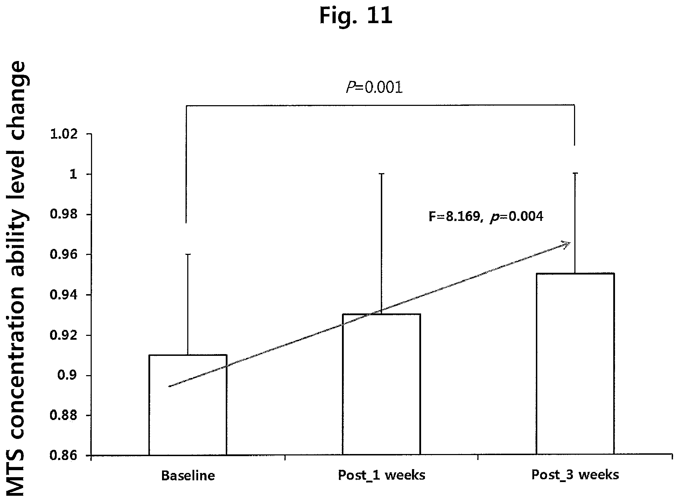

According to still another embodiment of the present invention, the present invention is directed to a functional beverage for improving cognitive functions. The intake of the functional beverage can improve cognitive functions, such as concentration ability, learning ability, and memory (see FIG. 11). In a particular embodiment, the improvement of the cognitive functions refers to the improvement of cognitive functions at the time of exercise.

According to still another embodiment of the present invention, the present invention is directed to a functional beverage having an anti-inflammatory effect. The intake of the functional beverage can suppress the inflammation responses (e.g., inflammation responses due to physical activity) (see FIGs. 14 to 16).

According to still another embodiment of the present invention, the present invention is directed to a functional beverage for maintaining immune functions or improving immune dysfunctions.

Excessive physical activity, such as long-term exercise, causes the deterioration of immune functions and the inflammation response due to physical stress. The blood IL-6 level, which is a cytokine related to the inflammation, the leukocyte count, and the hs-CRP level were significantly lower in the group drinking the functional beverage of the present invention than the non-drinking group (see FIGs. 14 to 16). These results show that the intake of the functional beverage of the present invention can improve immune dysfunctions and suppress the inflammation response.

In a particular embodiment, the physical activity includes ball sports, such as soccer and basketball, and aerobic exercises, such as walking, running (e.g., long-distance running, such as marathons or ultra-marathons), biking, hiking, swimming, and running on a treadmill.

The functional beverage of the present invention may be prepared in various forms. For example, the functional beverage of the present invention may be prepared in a form of drinking water, tea, sports drinks, ion beverages, or energy drinks. For example, when the beverage of the present invention is prepared as various drinks, the beverage may contain, in addition to high-hardness mineral water as an active ingredient, citric acid, high-fructose corn syrup, sugar, glucose, additional minerals (e.g., phosphate, iron, copper), various vitamins, acetic acid, malic acid, juice, various plant extracts (e.g., a plant extract containing polyphenol), and the like.

According to an embodiment of the present invention, a subject receiving the functional beverage of the present invention is a human being.

The mineral water of the present invention has a hard hardness, and is characterized by containing large quantities of magnesium, calcium, and potassium ions. However, the preparation of the mineral water containing such ions in large quantities has the following problems. As for a first problem, in cases where a mineral concentrate is added in large quantities to desalted water in order to increase the mineral content in the drinking water, the drinking water contains large quantities of sulfate and chlorine ions, which degrade the texture, as well as cations, such as magnesium or potassium ions. As for a second problem, in cases where calcium salts separated from concentrated water are added to desalted water in order to supply calcium, salt components and calcium carbonate contained in the calcium salts are added to degrade the texture of the drinking water, and due to the low solubility of calcium salts, the substantial supply efficiency of calcium is low. Therefore, the high-hardness mineral water is not easy to prepare due to the above problems.

Thus, the functional beverage of the present invention may include mineral water, which is prepared to have a hardness of 100-2,000 by mixing desalted water, which is obtained by performing a desalting treatment on salty underground water or deep ocean water, with: (i) a mineral concentrate obtained by separating calcium salt crystals and salt from concentrated water obtained by performing a desalting treatment on salty underground water or deep ocean water; and (ii) calcium salts obtained by removing, from the calcium salt crystals separated from the concentrated water, salt and calcium carbonate stuck onto the calcium salt crystals.

Respective steps will be described below.

The desalting treatment for separating desalted water and concentrated water from salty underground water or deep ocean water may be performed by employing any technique known in the art. Exemplary techniques for the desalting treatment may be an evaporation method, a seawater freezing method, a reverse osmosis method, an ion exchange resin method, an electrodialysis method, and the like. According to an embodiment of the present invention, through the desalting treatment, raw water is allowed to pass through a reverse osmotic membrane to be separated into concentrated water containing ion components and desalted water from which ion components are removed. The raw water may be used after having gone through a pretreatment process, such as filtration, for removing floating materials. The pretreatment process is conducted to remove impurities that may cause a membrane blockage in the reverse osmosis filtration, and typically, microfiltration or ultrafiltration may be conducted.

In the present invention, the desalted water may be obtained from the raw water by a desalting treatment with two or more steps. For example, the raw water is passed through a first reverse osmosis membrane to obtain first concentrated water and first desalted water, and the first desalted water is passed through a second reverse osmosis membrane to obtain second concentrated water and second desalted water. After that, calcium salts and a mineral concentrate, which are separated from the first concentrated water, are added to the second desalted water to prepare high-hardness mineral water.

The concentrated water contains calcium salts, such as calcium sulfate and calcium chloride, sodium chloride, and minerals in large quantities. In the present invention, the calcium salts and salts are gradationally separated from the concentrated water separated from the raw water.

In the present invention, for the precipitation of calcium salt crystals and salt from the concentrated water, the concentrated water is heated and concentrated such that the concentrated water has an appropriate specific gravity value (a ratio of concentrated water density over water density under the same temperature and pressure), thereby separating the precipitated calcium salt crystals and salt.

According to an embodiment of the present invention, for the separation of the calcium salts, the concentrated water is heated and concentrated such that the specific gravity (a ratio of concentrated water density over water density under the same temperature and pressure) is 1.11 or more, thereby separating the precipitated calcium salt crystals. The preferable specific gravity of the concentrated water for the separation of calcium salts is, but is not limited to, 1.11-1.23. The separation of calcium salts may be conducted using a mesh net, and the separation may be conducted using preferably a 300- to 350-mesh net, and more preferably a 300-mesh net.

According to another embodiment of the present invention, the concentrated water is heated and concentrated to have a specific gravity of 1.18, thereby first separating the precipitated calcium salts, and the filtrate is heated and concentrated to have a specific gravity of 1.19-1.23, thereby removing residual calcium salts.

According to still another preferable embodiment of the present invention, the concentrated water is heated such that it has a specific gravity of 1.23, and then is allowed to stand, thereby extracting calcium salts deposited on the bottom.

According to an embodiment of the present invention, the separation of the salt may be conducted, such that the concentrated water is heated and concentrated to have a specific gravity of 1.24 or more, thereby separating the precipitated salt. The preferable specific gravity of the concentrated water for the separation of the salt is, but is not limited to, 1.24-1.32. Since the mineral concentrate (liquid) and the salt (solid) are present together in the heated concentrated water, the salt may be separated by centrifugation or by using a dehydrator. The separated salt may be processed into mineral salt through an additional purification process.

In the present invention, the heating of the concentrated water may be slowly conducted simultaneously with stirring.

In the present invention, the concentrated water, from which the calcium salts and the salt have been removed, may be further heated and concentrated in order to concentrate mineral components.

According to an embodiment of the present invention, negative ions and impurities are removed from the mineral concentrate, from which calcium salts and salt components have been removed and which is mixed with desalted water, by a method for improving quality of the mineral concentrate, the method including: (a) filtering the mineral concentrate through a filter having a physical adsorbent; (b) re-filtering the filtered mineral concentrate through a filter having a physical adsorbent; and (c) filtering the mineral concentrate, which has been filtered in step (b), through a hollow fiber membrane filter having a plurality of pores.

The removal of the impurities is absolutely necessary for the preparation of drinking water, and the removal of negative ions is required to improve a feeling of refreshment of the mineral water. The present procedure is characterized by filtering the mineral concentrate through a filter having a physical adsorbent, and then re-filtering the filtered mineral concentrate through a filter having a physical adsorbent. For the filters used for the filtering and re-filtering, the same filter may be reused, or separate filters may be used. In addition, the filter for the re-filtering may be different from the filter used for first filtering with respect to the filter length, constituents, kind of adsorbent, and/or size of micropores of the adsorbent.

In the present invention, when the mineral concentrate passes through the filter having a physical adsorbent, negative ions (especially, chlorine ions and sulfate ions) and impurities (especially, silt) in the mineral concentrate are removed by being adsorbed on the plurality of micropores of the adsorbent, and the removing efficiency of negative ions and impurities is maximized through re-filtering using the filter having a physical adsorbent (first filtering). After that, the first-filtered mineral concentrated water is second filtered through a hollow fiber membrane filter, and thus, the removing efficiency of negative ions and impurities is further improved (second filtering).

In the present invention, any filter that has a physical adsorbent capable of adsorbing impurities and negative ions present in the solution may be used without limitation. According to an embodiment of the present invention, the physical adsorbent is activated carbon, diatomite, zeolite, silica gel, starch, bentonite, or alumina, and is more preferably activated carbon.

In the present invention, an appropriate activated carbon filter may be selectively used depending on the amount of the mineral concentrate. For example, for the treatment of 100 ℓ of the mineral concentrate, an 8- to 12-inch activated carbon filter is preferable, and for the treatment of 200 ℓ of the mineral concentrate, a 18-to 22-inch activated carbon filter is preferable. More preferably, a 10-inch activated carbon filter is used for treating 100 ℓ of the mineral concentrate, and a 20-inch activated carbon filter is used for treating 200 ℓ of the mineral concentrate.

In the present invention, the concentrated water, which has been first filtered through the filter having a physical adsorbent, is second filtered through a hollow fiber membrane filter, thereby further filtering out negative ions and impurities containing microorganisms and silt, and allowing the mineral components in the mineral concentrate to pass through the hollow fiber membrane filter. As the hollow fiber membrane filter, any micro-filter that has a plurality of pores may be used without limitation, and the pores have a diameter of 0.01-0.5 $\mu$m, preferably 0.05-0.5 $\mu$m, and more preferably 0.1-0.5 $\mu$m.

Preferably, the hollow fiber membrane filter is a sterilizing filter.

According to an embodiment of the present invention, the silt and negative ions in the mineral concentrate are removed by the filtering, and preferably, silt, chlorine ions, and sulfate ions are removed.

In the present invention, in order to improve the removing efficiency of impurities and negative ions, the circulation procedure may be repeatedly performed: filtering the mineral concentrate through a filter having a physical adsorbent → re-filtering the filtered concentrate through a filter having a physical adsorbent → re-re-filtering the re-filtered concentrate. The number of repetitions is preferably once or more, more preferably 1-5 times, and still more preferably 1-4 times.

In the present invention, the mineral concentrate may be supplied into the filter by a supply unit at an appropriate rate or an appropriate flow rate. The supply unit preferably employs a pump, and more preferably a controlled volume pump that can deliver the mineral concentrate to the filter at a constant rate (or flow rate).

According to an embodiment of the present invention, the removal of salt and calcium carbonate from the calcium salt crystals separated from the concentrated water may be conducted by (i) putting the calcium salt crystals, which has been separated from the concentrated water, into a container, which contains hot water and is equipped with a 300- to 350-mesh net; and (ii) separating the calcium salt crystals which do not pass through the net. Here, the salt stuck onto the calcium salt crystals is dissolved in the hot water, and the calcium carbonate passes through the mesh net and then is deposited on the bottom in the container. Since the calcium salt crystals separated from the concentrated water have been separated from the concentrated water containing large quantities of salt components, the calcium salt crystals necessarily contain the concentrated water. Therefore, in cases where the calcium salt crystals, without the removal of the salt components, are added to the desalted water, the salt component of the concentrated water degrades the texture of the mineral water. Moreover, the texture of the mineral water is degraded due to calcium carbonate. Thus, in the present invention, a purification procedure is performed to remove the calcium carbonate and salt (concentrated water) stuck onto the calcium salt crystals.

According to an embodiment of the present invention, in step (i), the container is slowly shaken after the calcium salt crystals are put into the container.

According to an embodiment of the present invention, for the mesh net in step (i), a net is used that has an equal or higher standard than the mesh net which has been used when the calcium salt crystals are extracted. More preferably, a 300-mesh (300 holes per inch) to 350-mesh net is used, and still more preferably, a 300-mesh net is used.

The temperature of the solution in the container is set to a temperature at which the calcium salts can be precipitated as crystals (the calcium salts can exist as crystals). When the temperature of the solution is lower than the above temperature, the calcium salts are dissolved in water, thereby lowering the filtering efficiency of calcium carbonate by the mesh net. The temperature of the solution may be set to 60-100°C, preferably 65-100°C, and more preferably 70-100°C. The reason is that, since the calcium salts are extracted at 70-100°C, the same conditions are set.

The calcium salt crystals separated in step (ii) may be dried by natural drying, a dry oven, a microwave oven, or the like, before being stored. Preferably, the calcium salt crystals are dried using a dry oven or a microwave oven.

After that, the stored calcium salts may be added to desalted water to supply calcium.

Through the above method, high-hardness mineral water can be prepared that contains large quantities of magnesium, calcium, and potassium and has an excellent texture, and the prepared high-hardness mineral water can be favorably used as a functional beverage.

Advantageous Effects

Features and advantages of the present invention are summarized as follows:

(i) The present invention relates to a functional beverage containing, as an active ingredient, high-hardness mineral water prepared from salty underground water or deep ocean water.
(ii) The functional beverage of the present invention can give a feeling of refreshment in the mouth if drank, effectively remove fatigue-inducing materials, such as lactate and reactive oxygen species, increase ATP production in the body to ultimately give vigor to daily life, and improve exercise ability and cognitive functions.
(iii) The functional beverage of the present invention can exhibit an anti-inflammatory effect and an effect of maintaining immune functions or improving immune dysfunctions.

Brief Description of the Drawings

FIG. 1 illustrates the cross-over design used in the test carried out in example 1.
FIG. 2 illustrates change in ATP level in the body for each group.
Group A: Control, Group B: Group drinking mineral water with a hardness of 300, Group C: Group drinking mineral water with a hardness of 700, Group D: Group drinking mineral water with a hardness of 1000
$p < 0.05$: *; $p < 0.01$: **; $p < 0.001$: *** (vs. Control)
$p < 0.05$: +; $p < 0.01$: ++ (vs. Group D)
FIG. 3 illustrates change in ROS level for each group.
Group A: Control, Group B: Group drinking mineral water with a hardness of 300, Group C: Group drinking mineral water with a hardness of 700, Group D: Group drinking mineral water with a hardness of 1000
FIGS. 4a to 4c illustrate changes in calcium, magnesium, and zinc levels in the whole blood for each group. Error bar represents SEM.
Group A: Control, Group B: Group drinking mineral water with a hardness of 300, Group C: Group drinking mineral water with a hardness of 700, Group D: Group drinking mineral water with a hardness of 1000
$p < 0.05$: *; $p < 0.01$: **; $p < 0.001$: *** (vs. Control)

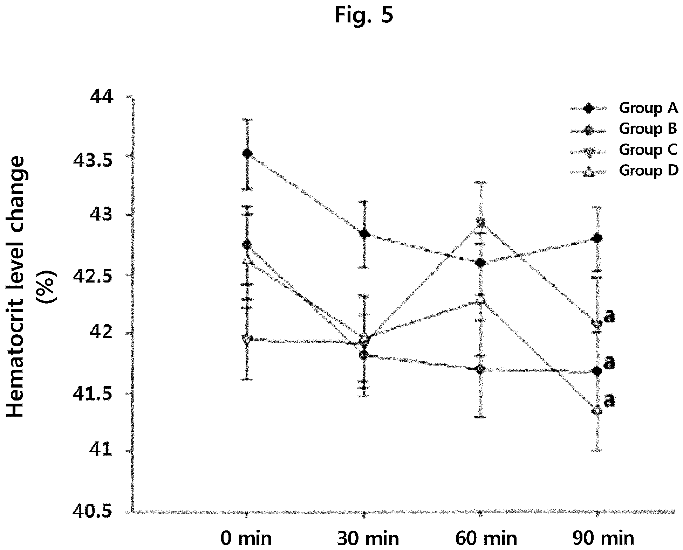

FIG. 5 illustrates the hematocrit level for each group. Error bars represent SEM.
Group A: Control, Group B: Group drinking mineral water with a hardness of 300, Group C: Group drinking mineral water with a hardness of 700, Group D: Group drinking mineral water with a hardness of 1000
a: $p < 0.005$ (vs. Control)

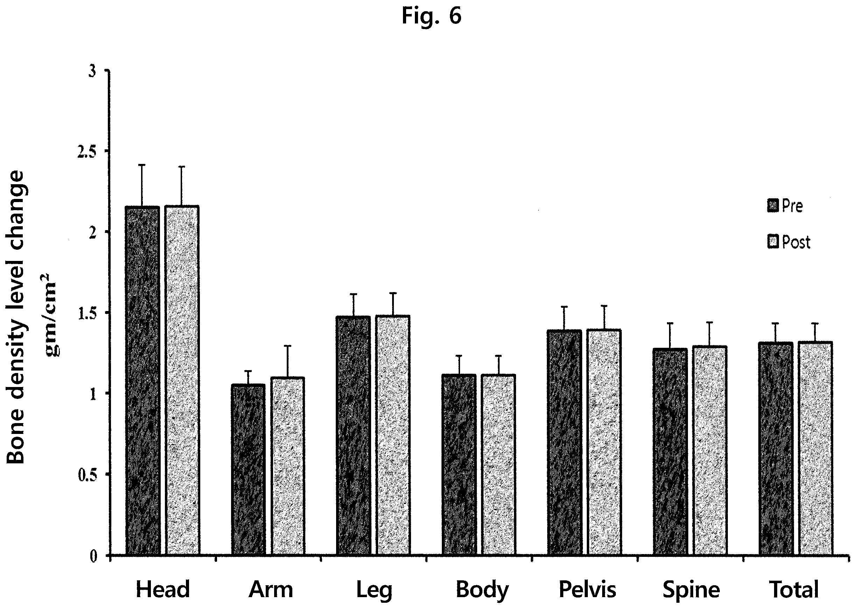

FIG. 6 illustrates the change in bone density for each part between pre- and post-intake of mineral water with a hardness of 700.

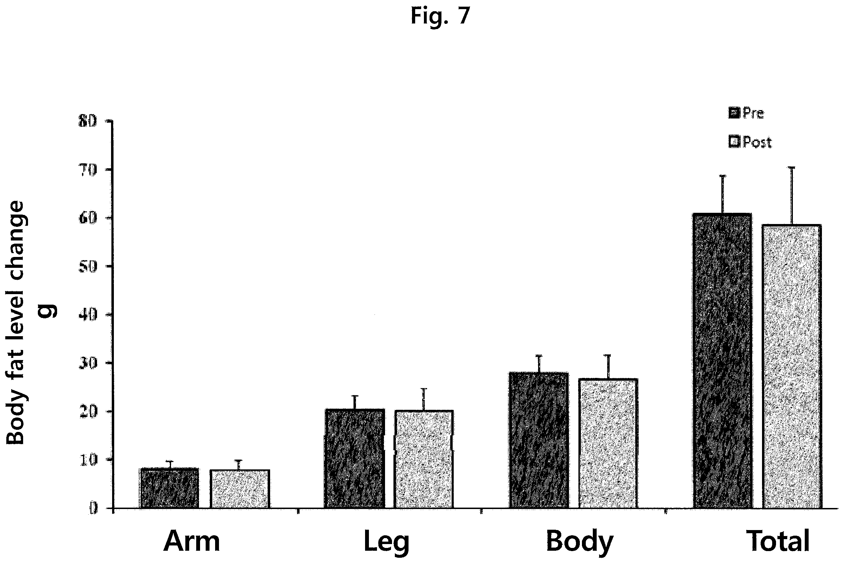

FIG. 7 illustrates the change in body fat for each part between pre- and post-intake of mineral water with a hardness of 700.

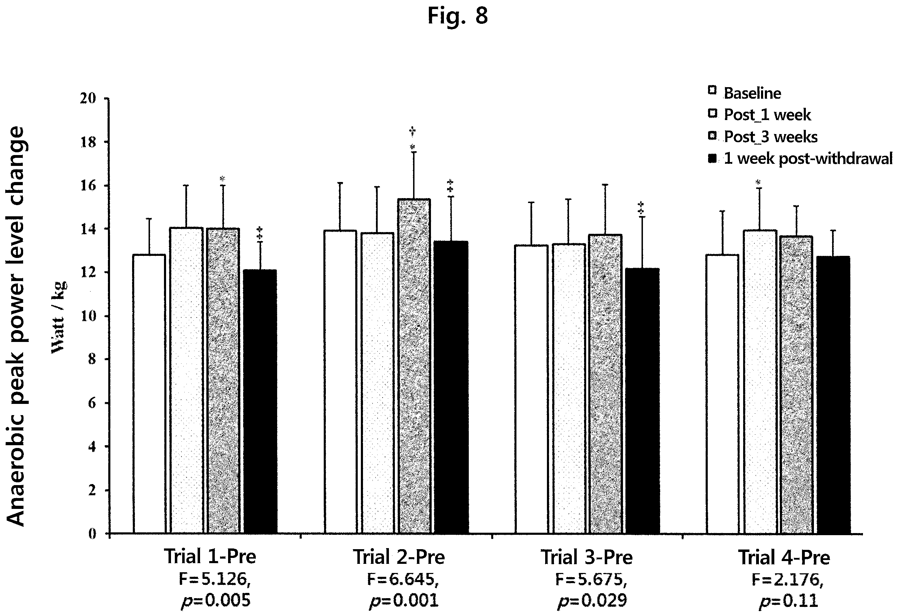

FIG. 8 illustrates the change in anaerobic peak power according to the intake of mineral water with a hardness of 700.
* $p < 0.05$, initial, 1 week post-intake, 3 weeks post-intake, and 1 week after withdrawal; † $p < 0.05$, 1 week and 3 weeks post-intake; ‡ $p < 0.05$, 3 weeks post-intake and 1 week after withdrawal
FIG. 9 illustrates the change in anaerobic mean power according to the intake of mineral water with a hardness of 700.
* $p < 0.05$, initial, 1 week post-intake, 3 weeks post-intake, 1 week after withdrawal; ‡ $p < 0.05$, 3 weeks post-intake and 1 week after withdrawal
FIG. 10 illustrates the change in the blood lactate level according to the intake of mineral water with a hardness of 700.
* $p < 0.05$, initial, 1 week post-intake, 3 weeks post-intake, and 1 week after withdrawal
FIG. 11 illustrates the change in MTS concentration ability according to the intake of mineral water with a hardness of 700.

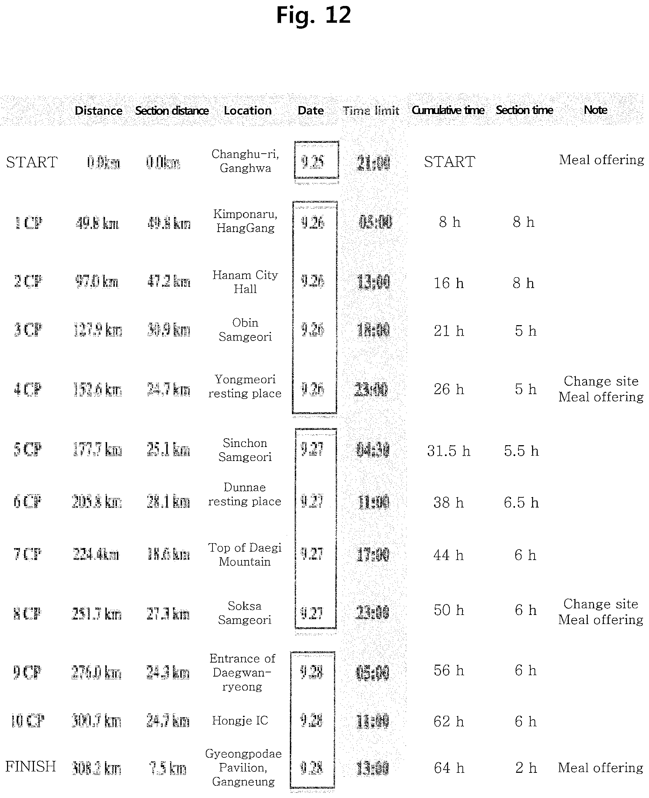

FIG. 12 schematically illustrates schedules of the test carried out in example 3.

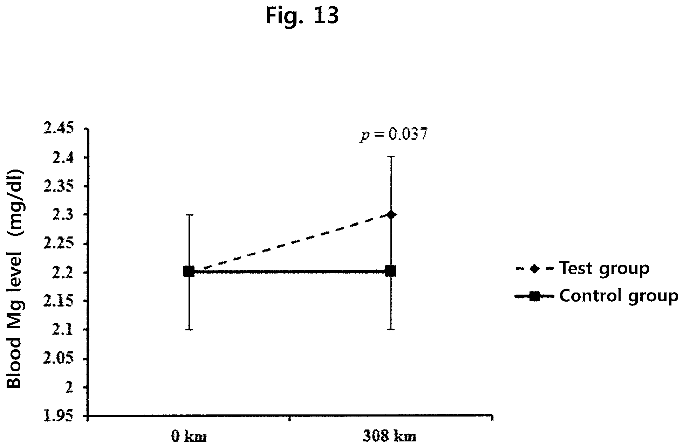

FIG. 13 illustrates the change in blood Mg level between pre- and post-308 km ultra-marathon according to the presence or absence of intake of mineral water with a hardness of 700.
FIG. 14 illustrates the change in blood IL-6 level between pre- and post-308 km ultra-marathon according to the presence or absence of intake of mineral water with a hardness of 700.

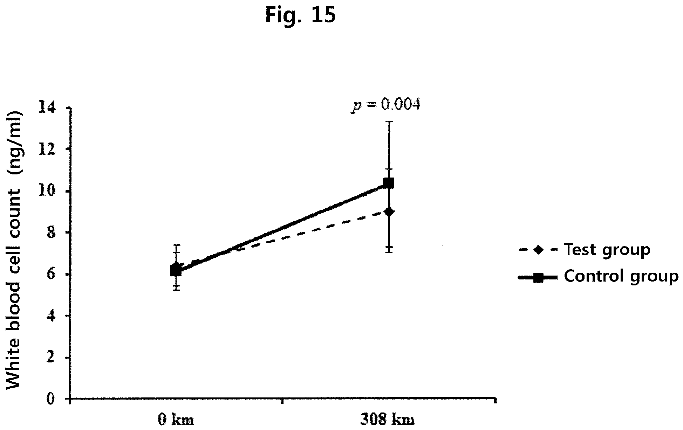

FIG. 15 illustrates the change in white blood cell count between pre- and post-308 km ultra-marathon according to the presence or absence of intake of mineral water with a hardness of 700.

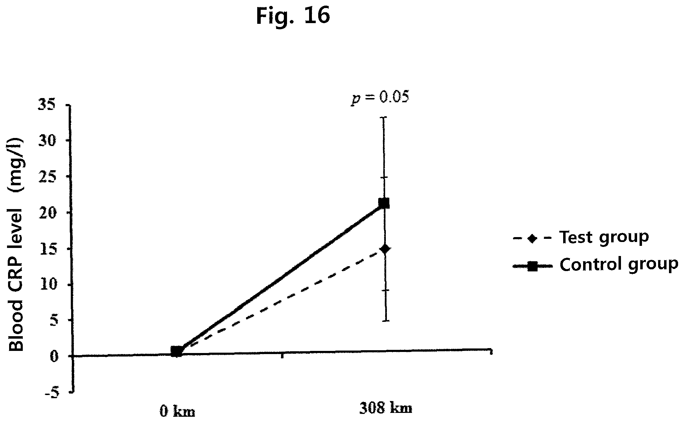

FIG. 16 illustrates the change in blood hs-CRP level between pre- and post-308 km ultra-marathon according to the presence or absence of intake of mineral water with a hardness of 700.

Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

**EXAMPLES**

**Example 1: Verification on increased ATP production and energy metabolism in body, reduced reactive oxygen species in body, and increased blood mineral level by intake of high-hardness mineral water 1-1. Methods**

*Subjects*

In the test, 24 students from the College of Physical Education, Yongin University, who signed consent forms with voluntary participation, were randomly allocated into four groups, 6 students in each group. Six students allocated to the control group were supplied with ordinary mineral water, and the treatment groups were supplied with mineral water with a hardness of 300, 700, or 1000 (Aribio Inc.).

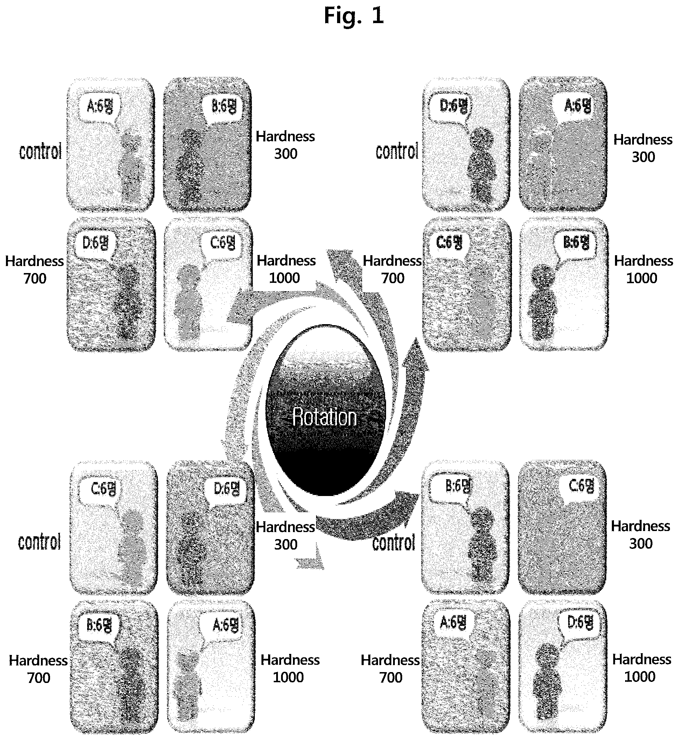

The test was carried out through a crossover design, using a repetitive measurement such that, after a washout period of 3-4 days following the intervention, the subjects were allocated to different treatment groups (FIG. 1). For the treatment, blood was taken post-intake of the drinking water assigned to each group on an empty stomach after waking up in the morning, and blood was taken in stable condition, 30, 60, and 90 minutes post-intake of the drinking water. The present study was carried out after the approval by Institutional Review Board (IRB) of Yongin University.

*ATP measurement*

The blood ATP level was measured using the ENLITEN ATP Assay system (Promega, WI, USA) according to the manufacturer's indication. Briefly, 100 $\mu\ell$ of the whole blood was put in 1 m$\ell$ of 0.5% trischloroacetate (TCA), incubated for 30 minutes at room temperature or on ice, and centrifuged at 15,000 RCF for 10 minutes. After the neutralization with 250 mM tris-acetate, the supernatant was diluted with 250 mM tris-acetate buffer, and then 10 $\mu\ell$ was dispensed in each well of the 96-well plate. Then, 100 $\mu\ell$ of a luciferase solution was put therein, followed by measurement using a luminometer (Infinite 200 pro, Tecan, Switzerland).

*ROS measurement*

In order to measure reactive oxygen species (ROS) scavenging ability, 2',7'-dichlorofluorescein diacetate (DCFDA) was used. White blood cells were separated from the whole blood through a density degradation method using Ficoll (Sigma, MO, USA), dispensed into a medium with 20 $\mu$M DCFDA added thereto, incubated at 37°C for 30 minutes, and then measured using the Tali image-based cytometer (Invitrogen, CA, USA).

*Statistical analysis*

For statistical analysis, continuous data were expressed as the mean and standard deviation. The analysis of the difference was made using SPSS statistical package 21 (SPSS Inc., Chicago, IL, USA), and the significance of the difference between groups was determined using repeated measure ANOVA test, and the Fisher's least significant difference test was used as a post hoc test for separating mean values. All statistic analyses were performed within a significance level of 5%.

**1-2. Results**

*Blood ATP analysis*

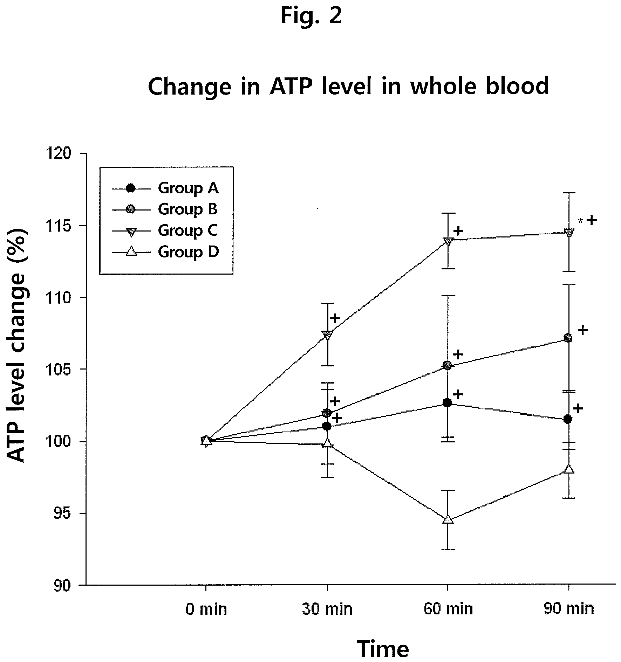

As shown in table 1 and FIG. 2, as a result of measuring ATP changes 30, 60, and 90 minutes post-intake, the increase in the ATP level was confirmed in the group drinking mineral water with a hardness of 300 and the group drinking mineral water with a hardness of 700, and especially, the ATP increase was higher in the group drinking mineral water with a hardness of 700.

[Table 1]

| Time Group | 0 min (% mean ± SEM) | 30 min (% mean ± SEM) | 60 min (% mean ± SEM) | 90 min (% mean ± SEM) |
|---|---|---|---|---|
| Group A | 100.00 ± 0.00 | 100.96 ± 2.59 | 102.55 ± 2.64a | 101.40 ± 2.04a |
| Group B | 100.00 ± 0.00 | 101.86 ± 2.17 | 105.17 ± 4.90a | 107.05 ± 3.74a |
| Group C | 100.00 ± 0.00 | 107.41 ± 2.17 | 113.89 ± 1.94a | 114.45 .± 2.73a,b |
| Group D | 100.00 ± 0.00 | 99.74 ± 2.29 | 94.44 ± 2.05 | 97.91 ± 1.96 |

a: $p < 0.05$ vs. group D; b: $p < 0.05$ vs. control group
Group A: Control, Group B: Group drinking mineral water with a hardness of 300, Group C: Group drinking mineral water with a hardness of 700, Group D: Group drinking mineral water with a hardness of 1000

*Reactive oxygen species (ROS) analysis*

As a result of measuring reactive oxygen species for each group post-intake of mineral water, as shown in table 2 and FIG. 3, the control group showed a highest increase in ROS level; the group drinking mineral water with a hardness of 300 showed a general decrease in ROS level; and the group drinking mineral water with a hardness of 700 and the group drinking mineral water with a hardness of 1000 showed remarkable decreases in ROS level.

[Table 2]

|  | 0 min | 30 min | 60 min | 90 min |
|---|---|---|---|---|
| Group A | 0 | -13.57 | 4.33 | 10.64 |
| Group B | 0 | -1.55 | -0.2 | 2.3 |
| Group C | 0 | -0.05 | 1.5 | -0.45 |
| Group D | 0 | -7 | -2.96 | -12.78 |

*Blood mineral analysis*

As shown in FIGs. 4a-4c, the blood calcium, magnesium, and zinc levels were increased from 30 minutes post-intake in all treatment groups compared with the control group.

As for calcium, compared with the level pre-intake, the levels at 30, 60, and 90 minutes post-intake showed: 0.26 %p, 0.33 %p, and 0.44 %p decreases, respectively, in the control group; 5.13 %p, 3.76 %p, and 1.70 %p increases, respectively, in the group drinking mineral water with a hardness of 300; 7.79 %p, 6.79 %p, and 4.93 %p increases, respectively, in the group drinking mineral water with a hardness of 700; and 10.41 %p, 9.57 %p, and 6.80 %p increases, respectively, in the group drinking mineral water with a hardness of 1000, and thus, there was a significant difference between groups ($p < 0.001$; FIG. 4a).

As for magnesium, compared with the level pre-intake, the levels at 30, 60, and 90 minutes post-intake showed: 0.50 %p and 0.67 %p decreases, and 0.25 %p increase, respectively, in the control group; 8.94 %p, 6.62 %p, and 2.91 %p increases, respectively, in the group drinking mineral water with a hardness of 300; 13.66 %p, 11.17 %p, and 7.10 %p increases, respectively, in the group drinking mineral water with a hardness of 700; and 26.77 %p, 20.26 %p, and 13.79 %p increases, respectively, in the group drinking mineral water with a hardness of 1000, and thus, it was analyzed that there was a level difference due to the intake ($p < 0.001$; FIG. 4b).

As for zinc, compared with the level pre-intake, the levels at 30, 60, and 90 minutes post-intake showed: 0.61 %p, 0.61 %p, and 0.74 %p increases, respectively, in the control group; 10.91 %p, 9.17 %p, and 6.26 %p increases, respectively, in the group drinking mineral water with a hardness of 300; 18.89 %p, 17.23 %p, and 12.12 %p increases, respectively, in the group drinking mineral water with a hardness of 700; and 24.07 %p, 20.39 %p, and 18.26 %p increases, respectively, in the group drinking mineral water with a hardness of 1000, and thus, there was a significant difference between groups ($p < 0.001$; FIG. 4c).

*Blood clinicopathologic analysis*

As shown in FIG. 5, as a result of blood clinicopathologic analysis, the hematocrit showed, as compared with the control group, a $0.95\pm0.27\%$ decrease in the group drinking mineral water with a hardness of 300, a $0.71\pm0.27\%$ decrease in the group drinking mineral water with a hardness of 700, and $0.89\pm0.27\%$ decrease in the group drinking mineral water with a hardness of 1000, and thus there was a significant difference ($p<0.05$).

**Example 2: Verification on reduced fatigue, improved exercise ability, and improved cognitive functions by intake of high-hardness mineral water 2-1. Methods**

*Study design*

Male boxers (n=9) and wrestlers (n=9) in Yongin University, Gyeonggi-do, participated in the present study, and the participants are athletes in physical matches in which muscular contraction form is intermittent and explosive force is instantly exerted. The study was performed in a repeated measure (RM) design, and there was a 1-week rest period before administration and after the baseline test. Following one week, the subjects were allowed to drink approximately 1 L (about 330 m$\ell$ for each breakfast, lunch, and dinner) of mineral water with a hardness of 700 (Aribio Inc.), and then the exercise function test was performed. After three weeks, a second test was performed. After all administrations were completed, the administration was withdrawn for one week, and then the exercise function test was performed.

Prior to the present measurement, it was checked whether the participants were normal or abnormal in muscular/skeletal and breathing/circulation systems, and then only subjects without medical findings were sorted. The subjects were made fully aware of the test schedule before the present study. In addition, the present study was approved by Institutional Review Board (IRB) under Yongin University.

*Dual Energy X-ray Absorptiometry (DEXA)*

The bone density and total bone density for each part were measured using dual energy X-ray absorptiometry (DEXA)-based Hv-ps 7681(GE medical systems Lunar, USA) before and after administration (3 weeks after). In order to prevent the occurrence of error in the radiation transmission rate, various kinds of metals (neckline, watch, etc.) were removed before radiographing.

*Anaerobic power measurement*

As for the anaerobic power measurement, the seat height and crank length were measured using an electromagnetic ergometer (computer-aided electrically braked cycle ergometer; Lode B. V. Excalibur Sports, Netherlands) after the explanation of the measurement procedure. The warm-up before the present measurement was performed at 60 rpm and 100 W for an initial 5 minutes such that the heart rate was maintained at at least 120-125 beat/min, and following a rest for 2 minutes, the present measurement was performed. The recovery was performed for 5 minutes after the first measurement, and second, third, and fourth measurements were performed continuously and repeatedly.

The Wingate test according to the intake of mineral water with a hardness of 700 was performed a total of four times, including before intake, one week after intake, three weeks after intake, and one week after withdrawal. The load was applied at 0.075 kp per body weight for the measurement, and in the entire measurement procedure, the time and intensity were adjusted by a computer using Lode Wingate version 1.0.7 software (Lode B.V., Netherlands). The loading level was constantly applied for the measurement, and verbal encouragement was made as much as possible for uniform psychological conditions during the measurement. As analysis factors, peak power/kg and mean power/kg were analyzed.

*Lactate level measurement*

The blood lactate level was measured in stable condition, immediately after first, second, and third Wingate exercises, pre-intake of mineral water, one week and three weeks post-intake of mineral water, and one week post-withdrawal. In order to measure the blood lactate level in the subjects, the blood was taken from a finger capillary vessel into a heparin-treated capillary tube using an instant blood collection device, and was analyzed using an automatic lactate analyzer (YSI 1500, U.S.A.). As a membrane kit necessary for the analysis, YSI 2329 (YSI Life Sciences) was used, and as a buffer, a mixture of purified YSI 2357 and 500 m$\ell$ of purified water was used.

*Cognitive function (concentration ability) test*

The concentration ability test for the athletes was evaluated as Match to Sample Visual Search (MTS) scores using Cambridge Neuropsychological Test Automated Battery (CANTAB, UK). For the measurement, the athletes rested for 30 minutes in a test room, and then the test was conducted in stable condition. Following the baseline test in stable condition, a Wingate anaerobic power test was conducted, and, immediately after the test, MTS re-evaluation was conducted. For all the measurements, evaluation was conducted pre-administration of mineral water, one week post-administration, three weeks post-administration, and one week post-withdrawal, thereby observing the change of concentration ability. The time for MTS test was about 6-10 minutes, and since the logical measurement, such as question response, was conducted according to personal competence, there were individual differences in the measurement time. The concentration ability test using MTS was selected for the present measurement since it is assessed to have high reproducibility, reliability, and validity compared with the questionnaire method that was used in existing brain function tests, and it is recently regarded as a valid evaluation method among brain health-related studies.

**2-2. Results**

*Change of Bone density for each part*

As a result of evaluating the change in bone density and the change in body fat for each part of the athletes pre-administration of mineral water with a hardness of 700 and 3 weeks post-administration thereof, there was mean quantitative increases in the bone density post-administration compared with pre-administration (FIG. 6), and the body fat also showed an average decrease trend in post-administration compared with pre-administration (FIG. 7).

*Anaerobic power test*

The Wingate anaerobic test according to the intake of mineral water with a hardness of 700 was performed a total of four times, including before intake, one week after intake, three weeks after intake, and one week after experiment completion. The test was repeatedly conducted four times for each test. The measurement was conducted five minutes after the first warm-up step, and second, third, and fourth measurements were conducted stage by stage 5 minutes after recovery. As test factors, a peak power, which is used as an index of explosive muscular strength, and a mean power, which is the maximum power generation ability for a 30-second section, were checked.

As a result of the change in peak power according to the intake of mineral water with a hardness of 700, as shown in FIG. 8, in the first Wingate test, the peak power was significantly increased (p=0.045) three weeks post-administration compared with the baseline, and significantly decreased (p=0.003) one week post-withdrawal compared with three weeks post-administration. As a result of the second Wingate test, the peak power was significantly increased (p=0.005) three weeks post-administration compared with the baseline, and significantly increased (p=0.025) three weeks post-administration compared with one week post-administration. In addition, the peak power was significantly decreased (p<0.0001) one week post-withdrawal compared with three weeks post-administration. As a result of the third Wingate test, the peak power showed an average increase trend one week and two weeks post-administration compared with the baseline. However, the peak power was significantly decreased (p=0.017) one week post-withdrawal compared with three weeks post-administration. As a result of the fourth Wingate test, the peak power was significantly increased (p=0.041) one week post-administration compared with the baseline.

As for the change results in mean power due to the intake of mineral water with a hardness of 700, as shown in FIG. 9, in the first and second Wingate tests, the mean power was significantly decreased one week post-administration compared with the baseline (first, p=0.003; second, p=0.03) and three weeks post-administration (first, p<0.0001; second, p=0.006). As a result of the third Wingate test, the mean power was significantly decreased (p=0.041) one week post-withdrawal compared with the baseline.

Through the change results in the anaerobic power according to the intake of the high-hardness mineral water, it was confirmed that the drinking for about three weeks is needed in order to significantly increase the peak power, and in a repeated power aspect, the drinking for about one week maximized the efficiency of the contribution ratio of the ATP-PC system. The contribution to the glycolysis system, represented by the mean power, showed a significant increase even three weeks post-administration, but a significant reduction at the withdrawal post-administration compared with the base line, and thus it is considered that the supply of the high-hardness mineral water maintains the glycolysis system.

*Change in blood lactate level*

The change in the blood lactate level was observed immediately after the Wingate test to perform evaluation on the basis of a delta value that was increased compared with when in stable condition. The time points for the blood lactate level test were immediately after the first Wingate test, and immediately after exercise for second, third, and fourth measurements, and the measurement was repeatedly conducted through the blood collection from the fingertip on the basis of the stable time. The comparison of the change in the blood lactate level was conducted pre-administration, one week post-administration, three weeks post-administration, and one week post-withdrawal.

As for the change results in the blood lactate level according to the intake of mineral water with a hardness of 700, as shown in FIG. 10, as a result of comparing the difference value from the level in stable condition immediately at the first Wingate test, the blood lactate level showed a decrease trend compared with pre-administration. In addition, as for the increase in the blood lactate level according to the second Wingate test, the lactate level was significantly decreased one week post-administration and one week post-withdrawal.

As a result of indirectly investigating the change of muscle buffering capacity after anaerobic exercise through the change in the blood lactate level as described above, it was confirmed that the accumulation of fatigue is reduced due to the intake of the high-hardness mineral water, thereby increasing the time for exercise.

*Concentration ability*

For the test of MTS concentration ability according to the intake of mineral water with a hardness of 700, evaluation was conducted pre-administration, one week post-administration, and three weeks post-administration. In addition, the MTS change was observed from after when in stable condition to immediately after exercise.

As a result of MTS concentration ability according to the intake of high-hardness mineral water, as shown in FIG. 11, a significant increase due to the administration was confirmed, and there was a significant difference three weeks post-administration.

**Example 3: Verification on anti-inflammatory effect and immune dysfunction preventing effect by intake of high-hardness mineral water**

**3-1. Methods**

In the test, 83 men in their 50s voluntarily participated in the present test, and the test was conducted in the 308 km Korean peninsula ultra-marathon race starting Ganghwado Island to the Gyeongpodae Pavilion, Gangneung, on October 2014. Although the 308 km ultra-marathon race was held for four days and three nights, the subjects received a simple meal and rest in a relaxation place and then participated in this race with no sleep. The blood was taken twice at the starting place and at the arrival place of 308 km, and the blood was taken immediately after the blood pressure measurement. Physical properties of the subjects participating in the study were as follows.

[Table 3]

|  | Test group (n=45) | Control group (n=38) | p for trend |
|---|---|---|---|
| Age (year) | $53\pm7$ | $51\pm8$ | 0.24 |
| Height (cm) | $168.7\pm6$ | $170.5\pm5$ | 0.12 |
| Body weight (kg) | $68.11\pm6.21$ | $68.66\pm6.53$ | 0.70 |
| Exercise carrier | $107.73\pm56.96$ | $107.68\pm56.59$ | 0.99 |

Since all the age, height, body weight, and exercise carrier showed normal distribution with skewness and kurtosis within $\pm2$, the conditions for testing a difference in means were secured.

In order to clearly find out the usefulness of the high-hardness mineral water (hardness, 700, Aribio Inc.) in the prevention of muscle fatigue and the generation of inflammation in the present study, a strict double-blind test was conducted. For the control group, drinking water having the same taste and color as the high-hardness mineral water was used in the same container.

The supply of the high-hardness mineral water was operated at 10 CP starting from the start place. The blood pressure was measured at every 100 km section in consideration of safety of the subjects. FIG. 12 illustrates the entire test schedule.

**3-2. Results**

As for the blood Mg level change before and after the 308-km running according to the presence or absence of the intake of high-hardness mineral water during the ultra-marathon race, as shown in FIG. 13, the blood Mg level was, on average, decreased in the control group, but increased in the test group, and thus there was a significant difference (p=0.037) between the two groups.

As for the IL-6 level change before and after the 308-km running according to the presence or absence of the intake of high-hardness mineral water during the ultra-marathon race, as shown in FIG. 14, the blood IL-6 level was significantly increased after the running rather than before the marathon race in the control group, but the change in the mean in the blood IL-6 level was not great in the test group, and thus there was a significant difference (p<0.0001) between the two groups.

As for the change of white blood cells, as shown in FIG. 15, the increase in white blood cells compared with before the ultra-marathon was significantly high in the control group compared with the test group, and thus there was a significant difference between the two groups (p=0.004).

As for the C-reactive protein (CRP) change before and after the marathon running according to the intake of high-hardness mineral water during the 308-km ultra-marathon race, as shown in FIG. 16, the CRP level was significantly increased compared with before the participation in the ultra-marathon in both of the control group and the test group, but the CRP level as the result of the running was significantly high in the control group compared with the test group, and thus there was a significant difference (p=0.05) between the two groups.

As described above, the test group drinking the high-hardness mineral water showed significantly low IL-6 level, white blood cell (WBC) count, and hs-CRP level compared with the control group. To sum the entire results, it can be seen that, in view of the generation of inflammation and the immune dysfunctions due to the long-term physical fatigue, the intake of the high-hardness mineral water can effectively suppress inflammation responses and protect immune functions.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

**Claims**

1. A functional beverage containing, as an active ingredient, mineral water having a hardness value of 100-2,000 prepared from salty underground water or deep ocean water.

2. The functional beverage of claim 1, wherein the functionality of the functional beverage is selected from the group consisting of increasing ATP production in the body, increasing energy metabolism in the body, increasing antioxidative activity in the body, increasing minerals in the body, suppressing lactate accumulation, improving exercise ability, reducing or recovering from fatigue, preventing muscular pain, improving cognitive functions, having an anti-inflammation effect, and maintaining immune functions or improving immune dysfunctions.

3. The functional beverage of claim 1, wherein the mineral water contains 15-500 mg/$\ell$ magnesium, 5-170 mg/$\ell$ calcium, and 4.5-150 mg/$\ell$ potassium.

4. The functional beverage of claim 1, wherein the mineral water has a hardness value of 100-1,200.

5. The functional beverage of claim 4, wherein the mineral water contains 15-300 mg/$\ell$ magnesium, 5-102 mg/$\ell$ calcium, and 4.5-90 mg/$\ell$ potassium.

6. The functional beverage of claim 1, wherein the functional beverage is selected from the group consisting of drinking water, tea, sports drinks, ion beverages, or energy drinks.

7. The functional beverage of claim 1, wherein the mineral water is prepared to have a hardness of 100-2,000 by mixing desalted water, which is obtained by performing a desalting treatment on salty underground water or deep ocean water, with: (i) a mineral concentrate obtained by separating calcium salt crystals and salt from concentrated water obtained by performing a desalting treatment on salty underground water or deep ocean water; and (ii) calcium salts obtained by removing, from the calcium salt crystals separated from the concentrated water, salt and calcium carbonate stuck onto the calcium salt crystals, and wherein the mineral concentrate is further treated, before the mixing, by filtering the mineral concentrate through a filter having a physical adsorbent, re-filtering the filtered mineral concentrate through a filter having a physical adsorbent, and then filtering the re-filtered mineral concentrate through a hollow fiber membrane filter having a plurality of pores.

8. A method for improving body functions, the method comprising, administering, to a subject, a functional beverage containing, as an active ingredient, mineral water having a hardness value of 100-2,000 prepared from salty underground water or deep ocean water, wherein the improving of the body functions is selected from the group consisting of increasing ATP production in the body, increasing energy metabolism in the body, increasing antioxidative activity in the body, increasing minerals in the body, suppressing lactate accumulation, improving exercise ability, reducing or recovering from fatigue, preventing muscular pain, improving cognitive functions, having an anti-inflammation effect, and maintaining immune functions or improving immune dysfunctions.

9. A use of the mineral water of any one of claims 1 to 7 for preparing a functional beverage, the mineral water having a hardness value of 100-2,000 prepared from salty underground water or deep ocean water.

# Fig. 1

# Fig. 2

## Change in ATP level in whole blood

# Fig. 3

## ROS change

## Fig. 4a

### Change in calcium level in whole blood

## Fig. 4b

Change in maganesium level in whole blood

# Fig. 4c

## Change in zinc level in whole blood

**Fig. 5**

Fig. 6

Fig. 7

EP 3 130 232 A1

# Fig. 8

EP 3 130 232 A1

Fig. 9

Fig. 10

EP 3 130 232 A1

# Fig. 11

# Fig. 12

| | Distance | Section distance | Location | Date | Time limit | Cumulative time | Section time | Note |
|---|---|---|---|---|---|---|---|---|
| START | 0.0km | 0.0km | Changhu-ri, Ganghwa | 9.25 | 21:00 | START | | Meal offering |
| 1 CP | 49.8 km | 49.8 km | Kimponaru, HangGang | 9.26 | 05:00 | 8 h | 8 h | |
| 2 CP | 97.0 km | 47.2 km | Hanam City Hall | 9.26 | 13:00 | 16 h | 8 h | |
| 3 CP | 127.9 km | 30.9 km | Obin Samgeori | 9.26 | 18:00 | 21 h | 5 h | |
| 4 CP | 152.6 km | 24.7 km | Yongmeori resting place | 9.26 | 23:00 | 26 h | 5 h | Change site Meal offering |
| 5 CP | 177.7 km | 25.1 km | Sinchon Samgeori | 9.27 | 04:30 | 31.5 h | 5.5 h | |
| 6 CP | 205.8 km | 28.1 km | Dunnae resting place | 9.27 | 11:00 | 38 h | 6.5 h | |
| 7 CP | 224.4km | 18.6 km | Top of Daegi Mountain | 9.27 | 17:00 | 44 h | 6 h | |
| 8 CP | 251.7 km | 27.3 km | Soksa Samgeori | 9.27 | 23:00 | 50 h | 6 h | Change site Meal offering |
| 9 CP | 276.0 km | 24.3 km | Entrance of Daegwan-ryeong | 9.28 | 05:00 | 56 h | 6 h | |
| 10 CP | 300.7 km | 24.7 km | Hongje IC | 9.28 | 11:00 | 62 h | 6 h | |
| FINISH | 308.2 km | 7.5 km | Gyeongpodae Pavilion, Gangneung | 9.28 | 13:00 | 64 h | 2 h | Meal offering |

# Fig. 13

# Fig. 14

EP 3 130 232 A1

## Fig. 15

32

# Fig. 16

## INTERNATIONAL SEARCH REPORT

International application No.

**PCT/KR2015/003188**

### A. CLASSIFICATION OF SUBJECT MATTER

***A23L 2/38(2006.01)i, A23L 1/30(2006.01)i, A23L 1/304(2006.01)i***

According to International Patent Classification (IPC) or to both national classification and IPC

### B. FIELDS SEARCHED

Minimum documentation searched (classification system followed by classification symbols)
A23L 2/38; A61K 33/14; A61K 33/06; A23L 2/52; A23L 1/307; A23L 2/70; A23L 1/304

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: saline underground water, deep see water, hardness, mineral water, functional beverage, sports drink

### C. DOCUMENTS CONSIDERED TO BE RELEVANT

| Category* | Citation of document, with indication, where appropriate, of the relevant passages | Relevant to claim No. |
|---|---|---|
| X | KR 10-1003856 B1 (DONGGUK UNIVERSITY GYEONGJU CAMPUS INDUSTRY-ACADEMY COOPERATION FOUNDATION) 23 December 2010<br>See claims 4-5; figure 3b; paragraph [0067]; abstract. | 1-7,9 |
| X | KR 10-2009-0013594 A (WATERVIS CO., LTD.) 05 February 2009<br>See claims 1-4. | 1,3-7 |
| X | KR 10-2014-0013857 A (KYUNGPOOK NATIONAL UNIVERSITY HOSPITAL)<br>05 February 2014<br>See claims 1-2. | 1,3-7 |
| A | KR 10-0873841 B1 (CHANGJO BIOTECH INC.) 15 December 2008<br>See claims 1-3. | 1-7,9 |
| A | US 6884444 B1 (IKEGAMI, Yoshinari et al.) 26 April 2005<br>See claim 1. | 1-7,9 |

☐ Further documents are listed in the continuation of Box C. ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 JULY 2015 (27.07.2015) | **27 JULY 2015 (27.07.2015)** |

| Name and mailing address of the ISA/**KR** | Authorized officer |
|---|---|
| Korean Intellectual Property Office<br>Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (July 2009)

## INTERNATIONAL SEARCH REPORT

International application No.

**PCT/KR2015/003188**

---

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: **8**
   because they relate to subject matter not required to be searched by this Authority, namely:

   Claim 8 pertains to a method for treating muscle pain, inflammation, immune function, and the like in the body by administering chemical components to the human body, and thus it is considered that claim 8 pertains to a method for treatment of the human body. Therefore, claim 8 pertains to subject matter on which the International Searching Authority is not required to carry out an international search under the provisions of PCT Article 17(2)(a)(i) and PCT Rule 3

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (July 2009)

INTERNATIONAL SEARCH REPORT
Information on patent family members

| International application No. |
|---|
| **PCT/KR2015/003188** |

| Patent document cited in search report | Publication date | Patent family member | Publication date |
|---|---|---|---|
| KR 10-1003856 B1 | 23/12/2010 | KR 10-2009-0119630 A | 19/11/2009 |
| KR 10-2009-0013594 A | 05/02/2009 | KR 10-0911949 B1 | 13/08/2009 |
| | | WO 2009-017379 A2 | 05/02/2009 |
| | | WO 2009-017379 A3 | 23/04/2009 |
| | | WO 2009-017379 A4 | 04/06/2009 |
| KR 10-2014-0013857 A | 05/02/2014 | WO 2014-017689 A1 | 30/01/2014 |
| KR 10-0873841 B1 | 15/12/2008 | NONE | |
| US 6884444 B1 | 26/04/2005 | AT 311121 T | 15/12/2005 |
| | | AU 2000-88200 A | 29/08/2000 |
| | | AU 764290 B2 | 14/08/2003 |
| | | CA 2363458 A1 | 17/08/2000 |
| | | CA 2363458 C | 12/08/2008 |
| | | CN 00236702 C | 18/01/2006 |
| | | CN 100337857 A | 27/02/2002 |
| | | CN 100611151 A | 04/05/2005 |
| | | DE 69928699 D1 | 05/01/2006 |
| | | DE 69928699 T2 | 27/07/2006 |
| | | DK 1161886 T3 | 27/03/2006 |
| | | EP 1161886 A1 | 12/12/2001 |
| | | EP 1161886 A4 | 24/07/2002 |
| | | EP 1161886 B1 | 30/11/2005 |
| | | ES 2251247 T3 | 16/04/2006 |
| | | NZ 513398 A | 28/06/2002 |
| | | TW 536393 B | 11/06/2003 |
| | | WO 00-47064 A1 | 17/08/2000 |

Form PCT/ISA/210 (patent family annex) (July 2009)